(19) Europäisches Patentamt
European Patent Office
Office européen des brevets

(11) Veröffentlichungsnummer: **0 271 834 B1**

(12) # EUROPÄISCHE PATENTSCHRIFT

(45) Veröffentlichungstag der Patentschrift: **07.09.94**

(51) Int. Cl.⁵: **C07D 239/52**, C07D 239/56, C07D 239/58

(21) Anmeldenummer: **87118370.3**

(22) Anmeldetag: **11.12.87**

Die Akte enthält technische Angaben, die nach dem Eingang der Anmeldung eingereicht wurden und die nicht in dieser Patentschrift enthalten sind.

(54) **Verfahren zur Herstellung von Pyrimidinen.**

(30) Priorität: **16.12.86 DE 3642830**

(43) Veröffentlichungstag der Anmeldung: **22.06.88 Patentblatt 88/25**

(45) Bekanntmachung des Hinweises auf die Patenterteilung: **07.09.94 Patentblatt 94/36**

(84) Benannte Vertragsstaaten: **CH DE FR GB IT LI NL**

(56) Entgegenhaltungen: **EP-A- 0 024 200**

(73) Patentinhaber: **HOECHST SCHERING AGREVO GmbH**
**Miraustrasse 54**
**D-13509 Berlin (DE)**

(72) Erfinder: **Lachhein, Stephen, Dr.**
**Kiedricher Strasse 18**
**D-6238 Hofheim am Taunus (DE)**
Erfinder: **Willms, Lothar, Dr.**
**Schulstrasse 3**
**D-5416 Hillscheid (DE)**

**EP 0 271 834 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung von Pyrimidinen der Formel I

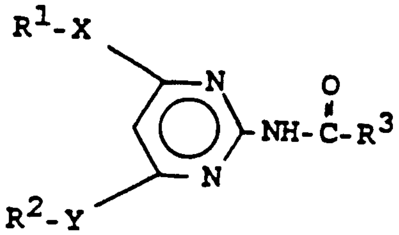

I,

worin

- X und Y: unabhängig voneinander Sauerstoff oder Schwefel,
- $R^1$ und $R^2$: unabhängig voneinander $(C_1\text{-}C_4)$Alkyl, $(C_1\text{-}C_4)$Alkoxy-$(C_1\text{-}C_2)$alkyl oder Halo$(C_1\text{-}C_4)$alkyl und
- $R^3$: Phenoxy, das durch ein, zwei oder drei Reste aus den Resten Halogen, $(C_1\text{-}C_4)$Alkoxy und $(C_1\text{-}C_4)$Alkyl substituiert sein kann, oder $(C_1\text{-}C_4)$Alkoxy bedeuten,

dadurch gekennzeichnet, daß man ein Propandiimidat der Formel II

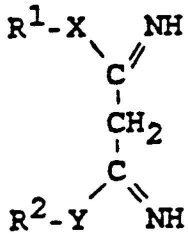

II,

worin $R^1$ und $R^2$ wie bei Formel I definiert sind, oder eines seiner Salze mit einem N-Cyanoamid der Formel III

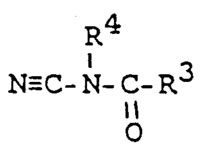

III,

worin

- $R^4$: H oder ein Kation bedeutet, in einem inerten Lösungsmittel umsetzt.

Verbindungen der Formel I sind wertvolle Zwischenprodukte bei der Herstellung von Sulfonylharnstoffen mit herbizider Wirkung (US-A-4,169,719, EP-A 071 958 (US-A-4,492,598)).

Bekannt ist die Herstellung der Verbindungen I über die entsprechenden Aminopyrimidine, wobei letztere durch Umsetzung von Propandiimidaten mit wäßriger Cyanamidlösung oder Chlorcyan in einem Zwei- oder Dreistufenprozeß hergestellt werden (EP-A-0 024 200). Die Bildung und anschließende Isolierung eines N-Cyanoimidates als Zwischenprodukt sind charakteristische Verfahrensmerkmale dieses Prozesses. Die dabei erhältlichen Ausbeuten für die Aminopyrimidine sind jedoch, unter anderem aufgrund einer nicht unerheblichen Menge an Nebenprodukten, unbefriedigend.

Überraschenderweise verläuft das erfindungsgemäße Verfahren demgegenüber als verfahrenstechnisch einfacher Einstufenprozeß ohne isolierbare Zwischenprodukte und liefert direkt die acylierten Verbindungen I. Nebenprodukte werden nur in untergeordnetem Maße gebildet. Nach Beendigung der Reaktion und anschließender Filtration verbleibt das Endprodukt in hoher Reinheit.

Beim erfindungsgemäßen Verfahren wird im Gegensatz zum obenbeschriebenen bekannten Verfahren kein N-Cyanoimidat als Zwischenstufe gebildet. Die hohe Selektivität bei der Bildung von Verbindungen der Formel I und die damit verbundenen hohen Ausbeuten waren nicht zu erwarten. Vielmehr war mit der Bildung von Nebenprodukten zu rechnen, da bekannt war, daß bei verwandten Reaktionen - der Umsetzung

von Guanyl-O-alkylisoharnstoffen anstelle von Imidaten mit Cyancarbonsäureestern - der Imidstickstoff ausschließlich mit der Carboxylgruppierung unter Triazinbildung reagiert (Chem. Pharm. Bull. 16 (3), 474 (1969)).

Das erfindungsgemäße Verfahren wird zweckmäßigerweise so durchgeführt, daß man das Propandiimidat als Mono- oder Disalz oder als Bisimidat isoliert, in Lösung oder als Suspension mit den N-Cyanamiden der Formel III bei Reaktionstemperaturen von 0-100 °C, vorzugsweise 10-80 °C, und pH-Werten zwischen 1-8, vorzugsweise 2-7, umsetzt. Als Salze des Propandiimidates werden bevorzugt solche der Fluor-, Chlor- oder Bromwasserstoffsäure, der Schwefel- oder Phosphorsäure eingesetzt. Haloalkylreste für $R^1$, $R^2$ sind beispielsweise $CH_2Cl$, $CHCl_2$, $CCl_3$, $CH_2CH_2Cl$ oder $CH_2CF_3$. Vorzugsweise bedeuten X und Y Sauerstoff. $R^1$, $R^2$ bedeuten vorzugsweise jeweils $(C_1$-$C_4)$Alkyl, insbesondere Methyl. Kationen für den Rest $R^4$ sind beispielsweise Alkali- und Erdalkalikationen, insbesondere $Na^+$, $K^+$, $\frac{1}{2}Mg^{2+}$.

Als inerte Lösungsmittel sind solche geeignet, die unter den jeweiligen Reaktionsbedingungen inert sind. Beispielsweise können als Lösungsmittel Wasser, Alkohole wie Methanol und Ethanol, Ketone wie Aceton und Methylisobutylketon, halogenierte Kohlenwasserstoffe wie Methylenchlorid und Chloroform, Ether wie Diethylether, Dioxan und Tetrahydrofuran, Ester wie Methylacetat, Ethylacetat und Butylacetat, Kohlenwasserstoffe wie Toluol, Xylol, Hexan und Cyclohexan, Nitrile wie Acetonitril, Säuren wie Ameisensäure und Eisessig oder deren Mischungen aus wenigstens zwei der aufgeführten Lösungsmittel verwendet werden.

Um störende Einflüsse vone Sauerstoff auf die Reaktion zu vermeiden, ist est zweckmäßig, unter Intergasatmosphäre, beispielsweise unter Stickstoff, zu arbeiten.

Die Verbindungen der Formel II können nach bekannten Methoden hergestellt werden (S.M. McElvain and I.D. Schroeder, JACS 71, 40 (1949); B. Harstun, DE-A-2 426 913). Das Monosalz und das Bisimidat dieser Verbindungen können aus dem entsprechenden Disalz durch Umsetzung mit Basen wie Alkali-une Erdalkalihydroxiden, -carbonaten, -hydrogencarbonaten oder -alkoholaten in einem unter den Reaktionsbedingungen inerten Lösungsmittel hergestellt werden.

Die Verbindungen der Formel III sind nach bekannten Methoden durch Umsetzung von Cyanamid mit dem entsprechenden Säurechlorid herstellbar.

Nachfolgende Beispiele sollen das erfindungsgemäße Verfahren näher erläutern:

**Beispiel 1**

154,8 g einer wäßrigen 50 %igen Cyanamidlösung in 300 ml Wasser werden unter Stickstoffüberlagerung gleichzeitig mit 179,4 g Chlorameisensäuremethylester und 586 g 25 %iger Natronlauge bei 40 °C und einem pH-Wert von 6,5-7,5 versetzt. Danach werden 315,0 g Dimethylpropandiimidatmonohydrochlorid zugegeben und die Mischung 2 Stunden bei 60 °C und einem pH-Wert von 5,0 gehalten. Nach Abkühlen auf Raumtemperatur wird mit 2n-Natronlauge auf pH 7 gestellt und abfiltriert. Die Ausbeute des getrockneten Produktes ist 366 g (≙ 91 % Ausbeute).
Der Schmelzpunkt beträgt 93-94 °C.

**Beispiel 2**

Zu einer Suspension von 70,4 g Natriumhydrogencarbonat in 920 ml Wasser werden langsam bei 0 °C 222,4 g Dimethylpropandiimidatdihydrochlorid gegeben. Zu dieser Mischung werden bei Raumtemperatur 520 ml einer Cyanamidcarboxylat-Lösung, hergestellt aus 96 g einer 50 %igen wäßrigen Cyanamidlösung und 121,2 g Chlorameisensäuremethylester, gegeben. Nach 3-stündigem Erhitzen auf 80 °C bei einem pH-Wert von 4 wird mit 2n-Natronlauge neutralisiert und abfiltriert. Es werden 209 g Produkt erhalten (≙ 90 % Ausbeute).
Der Schmelzpunkt beträgt 93-94 °C.

**Beispiel 3**

Zu einer Suspension von 222,4 g Dimethylpropandiimidatdihydrochlorid in 1000 ml Chloroform werden langsam bei 0 °C 231 g einer 50 %igen Natriumcarbonatlösung getropft. Zu diesem 2-Phasengemisch werden 520 ml einer Cyanamidcarboxylat-Lösung, hergestellt aus 96 g einer 50 %igen wäßrigen Cyanamidlösung und 121,9 g Chlorameisensäuremethylester, gegeben.
Diese Mischung wird für 4 Stunden auf 60 °C erhitzt, die Phasen getrennt und die organische Phase vom Lösungsmittel befreit.

Es verbleiben 172 g Produkt vom Schmelzpunkt 93-94 °C (≙ 90 % Ausbeute).

**Beispiel 4**

154,8 g einer wäßrigen 50 %igen Cyanamidlösung in 300 ml Wasser werden unter Stickstoffüberlagerung gleichzeitig mit 179,4 g Chlorameisensäuremethylester und 586 g 25 %iger Natronlauge bei 40 °C und einem pH-Wert von 6,5-7,5 versetzt. Danach werden 383 g Dimethylpropandiimidatdihydrochlorid bei 0 °C zugegeben und die Mischung für 3 Stunden bei 70 °C und einem pH-Wert von 5,0 gehalten. Nach Abkühlen auf Raumtemperatur wird mit Natronlauge auf pH 7 gestellt und abfiltriert.
Die Ausbeute des getrockneten Produktes beträgt 367 g (≙ 91 % Ausbeute).
Der Schmelzpunkt beträgt 93-94 °C.

Analog zu den in den Beispielen 1-4 beschriebenen Verfahrensweisen lassen sich folgende Verbindungen der Formel I herstellen:

| Beispiel | X | Y | $R^1$ | $R^2$ | $R^3$ |
|---|---|---|---|---|---|
| 5 | O | O | $CH_3$ | $CH_3$ | $OCH_3$ |
| 6 | O | O | $C_2H_5$ | $C_2H_5$ | $OCH_3$ |
| 7 | O | O | $C_2H_5$ | $C_2H_5$ | $CH_3$ |
| 8 | O | O | $CH_3$ | $CH_3$ | $O-C_6H_5$ |
| 9 | O | O | $C_2H_5$ | $C_2H_5$ | $O-C_6H_5$ |
| 10 | O | O | $CH_3$ | $CH_3$ | $OC_2H_5$ |
| 11 | S | S | $C_2H_5$ | $C_2H_5$ | $OCH_3$ |

| Beispiel | X | Y | $R^1$ | $R^2$ | $R^3$ |
|---|---|---|---|---|---|
| 12 | O | O | $CH-CH_3$<br>$\vert$<br>$CH_3$ | $CH-CH_3$<br>$\vert$<br>$CH_3$ | $OCH_3$ |
| 13 | O | S | $CH_3$ | $CH_3$ | $OCH_3$ |
| 14 | O | S | $C_2H_5$ | $C_2H_5$ | $OCH_3$ |
| 15 | O | O | $CH_3$ | $CH_3$ | $OC_2H_5$ |
| 16 | O | O | $C_2H_5$ | $C_2H_5$ | $OC_2H_5$ |
| 17 | O | O | $CH_2Cl$ | $CH_2Cl$ | $OCH_3$ |
| 18 | O | O | $CH_2Cl$ | $CH_2Cl$ | $OC_2H_5$ |
| 19 | O | O | $CH_2Cl$ | $CH_2Cl$ | $O-C_6H_5$ |
| 20 | O | O | $CF_3$ | $CF_3$ | $OCH_3$ |
| 21 | O | O | $CF_3$ | $CF_3$ | $OC_2H_5$ |
| 22 | S | S | $CF_3$ | $CF_3$ | $OC_2H_5$ |
| 23 | O | O | $CH_2OCH_3$ | $CH_2OCH_3$ | $OCH_3$ |
| 24 | O | S | $CH_2OC_2H_5$ | $CH_2OC_2H_5$ | $OCH_3$ |
| 25 | O | O | $CH_2OC_2H_5$ | $CH_2OCH_3$ | $OC_2H_5$ |

**Patentansprüche**

**1.** Verfahren zur Herstellung von Verbindungen der Formel I,

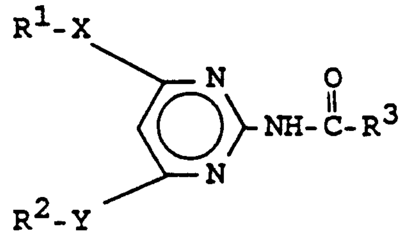

I,

worin

X und Y unabhängig voneinander Sauerstoff oder Schwefel,

$R^1$ und $R^2$ unabhängig voneinander $(C_1\text{-}C_4)$Alkyl, $(C_1\text{-}C_4)$Alkoxy-$(C_1\text{-}C_2)$alkyl oder Halo$(C_1\text{-}C_4)$-alkyl und

$R^3$ Phenoxy, das durch ein, zwei oder drei Reste aus den Resten Halogen, $(C_1\text{-}C_4)$Alkoxy und $(C_1\text{-}C_4)$Alkyl substituiert sein kann, oder $(C_1\text{-}C_4)$Alkoxy bedeuten,

dadurch gekennzeichnet, daß man ein Propandiimidat der Formel II

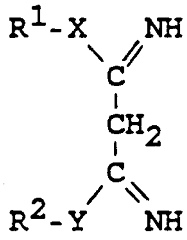

II

worin $R^1$ und $R^2$ wie bei Formel I definiert sind, oder eines seiner Salze mit einem N-Cyanoamid der Formel III

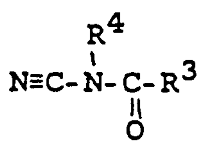

III,

worin

$R^4$ H oder ein Kation bedeutet, in einem inerten Lösungsmittel umsetzt.

**2.** Verfahren gemäß Anspruch 1, dadurch gekennzeichnet, daß in Formel I X und Y jeweils Sauerstoff und $R^1$, $R^2$ jeweils $(C_1\text{-}C_4)$Alkyl bedeuten.

**3.** Verfahren gemäß Anspruch 1 oder 2, dadurch gekennzeichnet, daß in Formel III $R^4$ = H bedeutet.

**4.** Verfahren gemäß einem oder mehreren der Ansprüche 1-3, dadurch gekennzeichnet, daß man als inertes Lösungsmittel Wasser, einen Alkohol, ein Keton, einen Kohlenwasserstoff, einen halogenierten Kohlenwasserstoff, einen Ether, einen Ester,ein Nitril, eine Carbonsäure oder eine Mischung aus wenigstens zwei der aufgeführten Lösungsmittel verwendet.

**5.** Verfahren gemäß einem oder mehreren der Ansprüche 1-4, dadurch gekennzeichnet, daß man als Lösungsmittel Wasser, Chloroform, Methanol, Aceton, Tetrahydrofuran, Acetonitril oder eine Mischung aus wenigstens zwei der Lösungsmittel verwendet.

6. Verfahren gemäß einem oder mehreren der Ansprüche 1-5, dadurch gekennzeichnet, daß man bei Reaktionstemperaturen von 0-100 °C arbeitet.

7. Verfahren gemäß einem oder mehreren der Ansprüche 1-6, dadurch gekennzeichnet, daß man bei Reaktionstemperaturen von 10-80 °C arbeitet.

8. Verfahren gemäß einem oder mehreren der Ansprüche 1-7, dadurch gekennzeichnet, daß man bei pH-Werten zwischen 1-8 arbeitet.

9. Verfahren gemäß einem oder mehreren der Ansprüche 1-8, dadurch gekennzeichnet, daß man bei pH-Werten zwischen 2-7 arbeitet.

10. Verfahren gemäß einem oder mehreren der Ansprüche 1-9, dadurch gekennzeichnet, daß man die Reaktion unter Inertgasatmosphäre durchführt.

## Claims

1. A process for the preparation of compounds of the formula I,

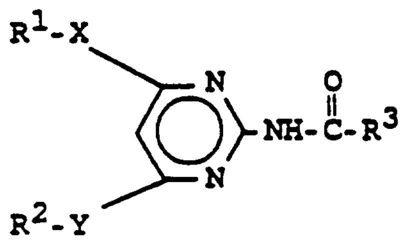

I,

in which

X and Y independently of one another, are oxygen or sulfur,

$R^1$ and $R^2$, independently of one another, are $(C_1\text{-}C_4)$alkyl, $(C_1\text{-}C_4)$alkoxy-$(C_1\text{-}C_2)$alkyl or halo $(C_1\text{-}C_4)$alkyl, and

$R^3$ is phenoxy which can be substituted by one, two or three radicals from the radicals halogen, $(C_1\text{-}C_4)$alkoxy and $(C_1\text{-}C_4)$alkyl, or is $(C_1\text{-}C_4)$alkoxy,

characterized by reacting a propanediimidate of the formula II

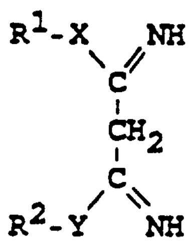

II

in which $R^1$ and $R^2$ are as defined under formula I, or a salt thereof,
with an N-cyanoamide of the formula III

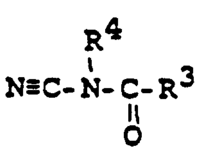

III,

in which

$R^4$ is H or a cation,

in an inert solvent.

**2.** The process as claimed in claim 1, wherein, in the formula I, X and Y are each oxygen and $R^1$ and $R^2$ are each ($C_1$-$C_4$)alkyl.

**3.** The process as claimed in claim 1 or 2, wherein, in the formula III, $R^4$ is H.

**4.** The process as claimed in one or more of claims 1-3, wherein the inert solvent used is water, an alcohol, a ketone, a hydrocarbon, a halogenated hydrocarbon, an ether, an ester, a nitrile, a carboxylic acid or a mixture of at least two of said solvents.

**5.** The process as claimed in one or more of claims 1-4, wherein the solvent used is water, chloroform, methanol, acetone, tetrahydrofuran, acetonitrile or a mixture of at least two of these solvents.

**6.** The process as claimed in one or more of claims 1-5, wherein the reaction is carried out at temperatures of 0-100 °C.

**7.** The process as claimed in one or more of claims 1-6, wherein the reaction is carried out at temperatures of 10-80 °C.

**8.** The process as claimed in one or more of claims 1-7, wherein the reaction is carried out at pH 1-8.

**9.** The process as claimed in one or more of claims 1-8, wherein the reaction is carried out at pH 2-7.

**10.** The process as claimed in one or more of claims 1-9, wherein the reaction is carried out under an inert-gas atmosphere.

**Revendications**

**1.** Procédé pour la préparation de composés de formule I

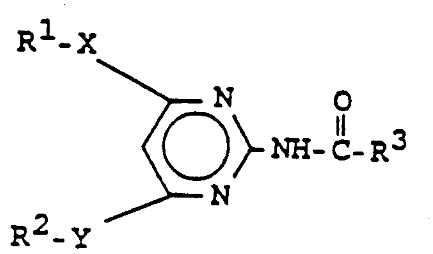

(I)

où

X et Y, indépendamment l'un de l'autre, signifient l'oxygène ou le soufre,

$R^1$ et $R^2$, indépendamment l'un de l'autre, les alkyle en ($C_1$-$C_4$), alcoxy en ($C_1$-$C_4$)-alkyle en ($C_1$-$C_2$) ou halogéno-alkyle en ($C_1$-$C_4$), et

$R^3$ le phénoxy qui peut être substitué par un, deux ou trois radicaux pris dans le groupe comprenant les radicaux halogène, alcoxy en ($C_1$-$C_4$) et alkyle en ($C_1$-$C_4$), ou signifie un alcoxy en ($C_1$-$C_4$),

caractérisé en ce qu'on fait réagir un propane-diimidate de formule II

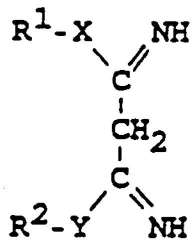

(II)

où $R^1$ et $R^2$ sont définis comme dans la formule I, ou un de ses sels avec un N-cyanamide de formule III

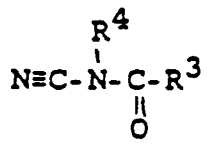

(III)

où

$R^4$ signifie H ou un cation, dans un solvant inerte.

**2.** Procédé selon la revendication 1, caractérisé en ce que dans la formule I, X et Y signifient chaque fois l'oxygène et $R^1$, $R^2$ chaque fois un alkyle en ($C_1$-$C_4$).

**3.** Procédé selon les revendications 1 ou 2, caractérisé en ce que dans la formule III $R^4$ signifie H.

**4.** Procédé selon une ou plusieurs des revendications 1 à 3, caractérisé en ce qu'on utilise en tant que solvant inerte l'eau, un alcool, une cétone, un hydrocarbone, un hydrocarbone halogéné, un éther, un ester, un nitrile, un acide carboxylique ou un mélange d'au moins deux des solvants indiqués.

**5.** Procédé selon une ou plusieurs des revendications 1 à 4, caractérisé en ce qu'on utilise en tant que solvant l'eau, le chloroforme, le méthanol, l'acétone, le tétrahydrofuranne, l'acétonitrile ou un mélange d'au moins deux des solvants.

**6.** Procédé selon une ou plusieurs des revendications 1 à 5, caractérisé en ce qu'on travaille à des températures de réaction de 0 à 100 °C.

**7.** Procédé selon une ou plusieurs des revendications 1 à 6, caractérisé en ce qu'on travaille à des températures de réaction de 10 à 80 °C.

**8.** Procédé selon une ou plusieurs des revendications 1 à 7, caractérisé en qu'on travaille à des du pH de 1 à 8.

**9.** Procédé selon une ou plusieurs des revendications 1 à 8, caractérise en ce qu'on travaille à des pH de 2 à 7.

**10.** Procédé selon une ou plusieurs des revendications 1 à 9, caractérisé en qu'on met en oeuvre la réactions sous une atmosphère de gaz inerte.